# EUROPEAN PATENT APPLICATION

(11) **EP 1 913 922 A2**
(43) Date of publication of application: **23.04.2008**
(21) Application number: 07020599.2
(22) Date of filing: 19.10.2007
(51) Int. Cl.: A61H 31/00

(54) **Chest compression system**

(30) Priority: 20.10.2006 US 584243; 20.10.2006 NO 20064769
(71) Applicant: Laerdal Medical AS, 4002 Stavanger (NO)
(72) Inventor: Stromsnes, Oystein, 4120 Tau (NO)
(74) Representative: HOFFMANN EITLE

(57) **Abstract**

The invention regards a resuscitation system which comprises chest compression devices (1) to repeatedly compress the chest of a patient and thereafter causing or allowing the chest to expand, power supply devices (20), and a signal processor (4) connected to the chest compression devices to control operation of the chest compression devices.

## Description

This invention is related to resuscitation systems.

Sudden cardiac arrest is a leading cause of death in developed countries in the Western World, like United States and Canada.

To increase the chance for survival from cardiac arrest, important aspects are CPR (Cardio Pulmonary Resuscitation) and heart defibrillation given in the first few critical minutes after the incident.

CPR is performed to ensure a sufficient flow of oxygenated blood to vital organs by external compression of the chest combined with rescue breathing. Heart defibrillation is performed to re-establish normal heart rhythm by delivery of an external electric shock.

The *quality* of CPR is essential for survival. Chest compressions must be given with a minimum of interruptions, and be of sufficient depth and rate. Performing chest compressions manually is an extremely exhausting task, and it is practically impossible to give manual CPR of sufficient quality during transportation of a patient.

To overcome this problem, a number of automatic and manual mechanical external chest compression devices for cardiopulmonary resuscitation have been developed.

Michigan Instruments has a device for automatic mechanical external chest compressions consisting of a vertical column attached to a base plate. The device is described in US 6171267 and US 5743864. A cantilevered arm with a cylinder and piston assembly slides up and down on a column to adjust the piston position to the patient. The system comprises a measuring device to measure the depth of the patient's chest (thorax). The depth may be compared to a table in order to adjust the compression depth to each patient. This leads to delay in start-up of the compressions and the procedure does not compensate for possible chest collapse during therapy. Compressed gas (oxygen or air) drives the device. The device may comprise a ventilator for ventilating the patient.

US 2003181834 describes another chest compression apparatus. The device comprises a back plate for positioning behind the patient's back posterior to the patient's heart and a front part for positioning around the patient's chest anterior to the patient's heart. The front part comprises two legs which can be coupled to the back plate. The front part comprises a compression unit which automatically compresses or decompresses (lifts) the patient's chest. The width and compression depth of the apparatus is fixed and cannot be adapted to each patient. This device is gas driven, which means that it is large and heavy to use due to the need of compressed gas. The compressed air may be substituted by compressed oxygen, for example when running out of gas, but this may lead to an increased risk of fire.

US 6398745 is another example of an automatic CPR-device. This device uses a compression belt around the chest of the patient which compression belt is repetitively tightened and relaxed through the action of a belt tightening spool powered by an electric motor. During use the compression belt will compress and decompress the chest and can easily get caught in the patient's clothes. For this reason the patient must be unclothed before the chest compression procedure can start, and valuable time is lost. Besides the belt covers a large area of the patient's chest and thus interferes with defibrillation electrodes (requiring that the defibrillator electrodes is arranged on the patient prior to the arrangement of the belt or that the belt is removed before defibrillation can take place) and makes stethoscope check of correct intubation and check of chest rise cumbersome. The motor is controlled by a control system which times the compressions and controls the compressions through an assembly of clutches and brakes connecting the motor to the belt spool.

The above mentioned devices have limitations in use, and even though automatic mechanical CPR is well documented to deliver adequate circulation to the brain and heart during CPR, such systems have not been taken widely into use by medical personnel. The reasons for this are among others that they either are complicated and time-consuming to apply, cumbersome to install and operate, or are unstable on the chest. They are further heavy and expensive to purchase.

The obj ects of the invention are to provide a resuscitation device which is easy to use, rugged, portable and light weight, safe and reliable, which has an intuitive user interface, ensures patient stability and has an affordable price.

These and other objects are achieved by means of a resuscitation system comprising chest compression devices to repeatedly compress the chest of a patient and causing or allowing the chest to expand, power supply devices, and a signal processor connected to the chest compression device to control operation of the chest compression device.

The chest compression device may be any device suitable for compressing the chest of a patient, such as pneumatic, hydraulic, or electric actuated pistons, belts, straps, etc. The chest compression device may be fixed to the patient's chest/skin by means of fastening devices such as tape or by vacuum, or it can be merely in contact with the chest without being fastened to the chest. The chest compression device can be designed to cause the chest to expand, i.e. is to perform an active lifting of the chest, or to allow the chest to expand freely.

Besides, the chest compression device comprises or is connected to a support in order to maintain a substantially constant positioning of the chest compression device on the patient's chest. A substantially constant positioning of the chest compression device on the patient's chest is important in order to obtain the necessary quality of the compressions and for safety reasons.

Said support may be adapted to be arranged under the patient, on one of the sides, or reaching round the patient.

In one embodiment, the support comprises two legs connected to a back plate and to a transverse plate. The back plate is adapted for placement under the patient when the patient is lying, and the two legs are adapted for placement on both sides of the patient. The width and the height of the quadrangle may be adjusted to fit each patient.

The support should be symmetric and surround the patient closely in order to give a stable support and ensure correct positioning of the compression device and thus the compressions on the patient.

In one embodiment of the invention the compression device is firmly and fixed connected to the transverse plate in a position which ensures correct positioning on the patient's chest. In an alternative embodiment, the compression device may constitute the transverse plate completely or partially. The compression device is connected to the support in such a way that the direction of the compression movement of the chest compression device is substantially perpendicular to thorax in the area between the nipples. This may for example mean that the movement of the compression device is substantially perpendicular to a plane comprising sternum, and substantially parallel to the back plate.

The positioning of the patient relative to the support is partly or fully a inherent property of the support. The positioning relative to the patient's width may be performed by arranging the legs of the support close in contact with the patient's sides in the embodiments of the support comprising legs. The positioning relative to the patient's length may be performed by means of illustrations on the support or by other display devices and supervised by the operator. In one embodiment the system may comprise physical devices or arrangements which indicates and/or guides the positioning of the system relative to the patient, for example by arranging the legs in the patient's armpits, by a rod indicating the distance to the patient's shoulders, etc.

In other embodiments, the support can be a frame, stand, rack, tripod, etc. of suitable design.

The support may be collapsible, demountable or foldable in order to minimize volume of the system when not in use. Preferably, the support is easy to assemble and prepare for use in order to minimize time wasted on assembling and mounting. This may for example be achieved by using spring-loaded elements which unfold themselves to the maximum size. The system should comprise as few separate parts as possible in order to minimize risk of incorrect assembly and to minimize assembly time. In one embodiment the system only comprises two separate sections for assembly.

The resuscitation system according to the invention comprises power supply devices. The power supply devices may comprise power storage devices such as batteries, for example Lithium-ion chemistry type batteries, or devices for connection to power sources in an ambulance, in a hospital, or in an external power storage device such as a battery or capacitor, or any other available power supply device. The resuscitation system may comprise power adapters/converters to be able to use power supplies with different characteristics/properties such as different voltage, frequency, etc.

The resuscitation system according to the invention comprises a processor connected to the chest compression device for providing control signals to the compression device. The control signals control the actuation of the chest compression device, and may be signals for controlling an electric motor. The control signals may be start/stop signals and/or signals controlling e.g. depth/force/frequency of the compressions. The control signals may for example be based on patient characteristics, such as a measured chest height/depth of the patient, age of the patient, ECG measurements, etc. In this way the resuscitation system may use a pulse pattern particularly adapted to the specific patient.

In one embodiment of the invention the system comprises measuring devices for measuring characteristics of the resuscitation process, and the signal processor is adapted to process input signals from the measuring devices.

The measuring device(s) may be any sensors or other measuring devices suitable for measuring characteristics of the resuscitation process, and other relevant information regarding CPR in the system and/or in the patient. Such sensors/measuring devices are for example force sensors and/or depth sensors for measuring force/depth exerted/traveled by the compression device, compression counters, compression frequency counters, blood flow sensors for monitoring the blood flow of the patient, ventilation sensors for monitoring the ventilation flow, volume, and/or time interval of patient ventilation, impedance measuring means for measuring the impedance of the chest and thus give an indication of the ventilation of the patient, electrocardiogram (ECG) device, tilt sensors for measuring the angle of the patient (whether the patient is lying, sitting/standing), position detectors for detecting the positioning and/or change of positioning of the chest compression means, battery power measurement means, internal motor temperature measuring means, etc. The results from the measuring devices may be used to provide information to the users and/or as feedback to the processor for adjusting/changing the control signals to the compression device.

The resuscitation system according to the invention may also comprise ventilation devices. The ventilation devices may be regular ventilation devices which may be operated by an operator, or they may be autonomous/automatic ventilation devices. In the case where the ventilation devices are operated by an operator, the resuscitation system may comprise a sensor for measuring characteristics (quality) of the ventilation, such as ventilation rate and volume. The resuscitation system may also comprise feedback devices such as speaker or display to give feedback to the operator on the performed ventilations, position stability of the chest compression device, time left of battery, stiffness changes in the patient's chest, or other aspects regarding system or patient.

The resuscitation system according to the invention may also comprise defibrillator devices in order to be able to defibrillate the patient. The defibrillator device may be manual or automatic (AED). The defibrillator devices may comprise ECG measuring devices. The system may also comprise a separate or integrated processor which can calculate the optimal point in time to operate the defibrillator device and the optimal characteristics of the defibrillation and start the defibrillator or give an indication on correct start time based on these values.

The resuscitation system according to the invention may further comprise data storing devices for storing values of the measured characteristics. These stored values may later be used for evaluating the resuscitation episode. In this way systematic or occasional operator errors may also be revealed and this knowledge may be used to adjust procedures and/or train personnel. Stored values may also be used to reveal equipment errors and initiate service.

The resuscitation system according to the invention may further comprise a user interface for providing information regarding the resuscitation to the helping personnel. The user interface may be comprised in or be separate from the feedback devices. The user interface may provide information related to the service level of the system, remaining power, defibrillator status, ventilation status or other information which would be useful for the operator during or after the resuscitation.

The invention will now be described in more detail by devices of examples and with reference to the attached figures, where:
Figure 1 is a block diagram of an embodiment of the invention,
Figure 2 is a perspective view of an embodiment of the invention.
Figure 3 is a perspective view of the embodiment of figure 2 in a folded state.

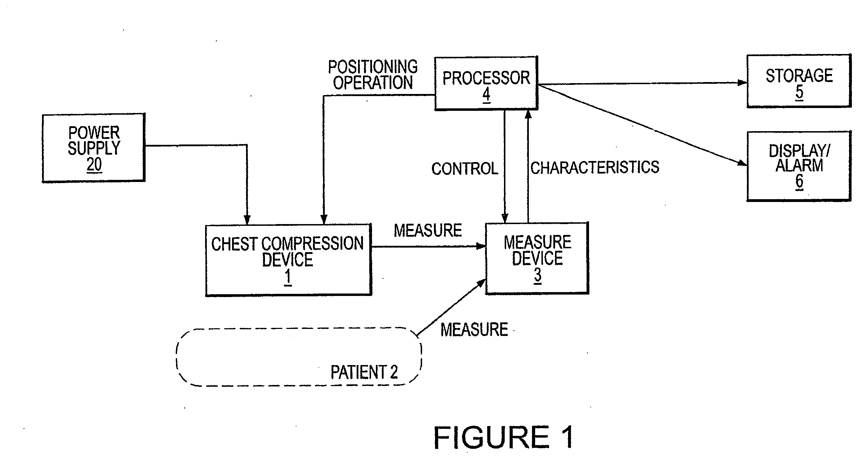

Figure 1 is a block diagram of an embodiment of the resuscitation system according to the invention. This block diagram shows chest compression devices 1 for repeatedly compressing the chest of a patient 2 and causing or allowing the chest to expand, measuring devices 3 for measuring characteristics of the resuscitation process, a power supply 20 and a signal processor 4 connected to the measuring devices 3 and/or the chest compression devices 1, to control operation of the chest compression devices 1. In this embodiment of the invention the signal processor 4 is connected to data storing devices 5 to permit storage of measurement values and thus provide historical data, and to display devices 6 to display characteristics of the resuscitation process, and/or alarms.

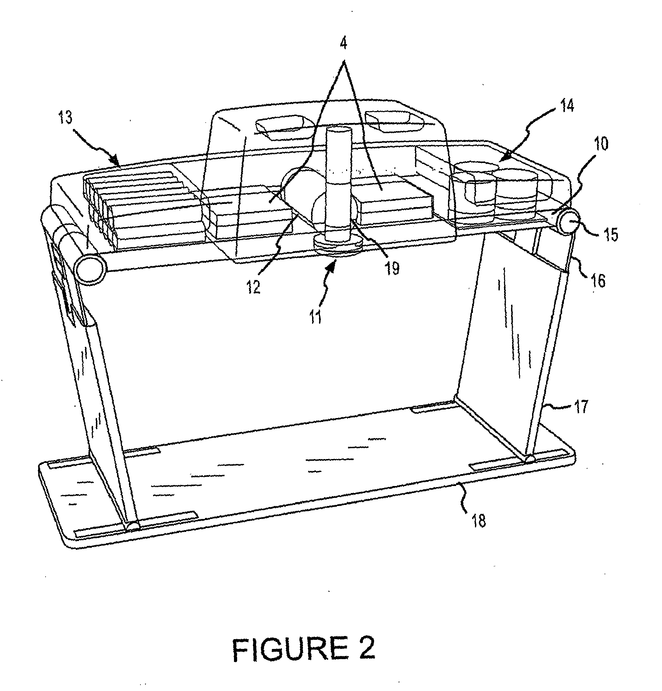

Figure 2 is a perspective view of an embodiment of the invention. In this embodiment of the invention, the chest compression devices 1, the signal processor 4 and the power supply are situated on a transverse plate 10.

The chest compression devices comprise in this embodiment of the invention a piston 11, a transmission mechanism 19 for transmitting energy to the piston 11, a motor 12. The power supply comprises batteries 13 and boost electronics 14. Piston 11 is in charge of performing compression and allowing or performing decompression of the patient's chest. The aim of the boost electronics is to provide a high energy, short pulse to the motor's 12 power input.

Signal processor 4 comprises in this embodiment of the invention devices for controlling operation of the chest compression devices based on predetermined characteristics and/or on characteristics measured by measuring devices (not shown). These characteristics can be, as mentioned before force exerted by the piston, depth traveled by the piston, compression frequency, blood flow in the patient, ventilation flow, etc.

The above mentioned devices are situated on a transverse plate 10. Transverse plate 10 is substantially rectangular and is connected on its short edges to two lateral legs. The connection between transverse plate 10 and the legs is implemented by hinges 15, this permitting to rotate the legs towards the transverse plate to provide a storage position for the resuscitation system. The legs comprise an upper part 16 and a lower part 17. The upper part 16 is hinged to the transverse plate 10 and is also situated telescopically inside the lower part 17. This arrangement permits easy variation of the legs' length. The legs are adapted for placement on the sides of the patient's body. The lower edge of the lower part 17 is connected to a back plate 18 adapted for placement under the patient's back. The legs may be moved laterally to closely surround the patient.

The legs' lower parts 17 can be fixed, shiftable, or rotatably connected to back plate 18. In one embodiment of the invention they are laterally shiftable in order to be able to be arranged in contact with the patient's body, and when in correct position they are fixed connected to plate 18. The legs may be separated from the back plate during transport and storage providing two separate sections, one comprising the transverse plate with the above mentioned devices on it and the legs, and one comprising the back plate 18. The separate sections can be folded to a flat position permitting easy storage of the device.

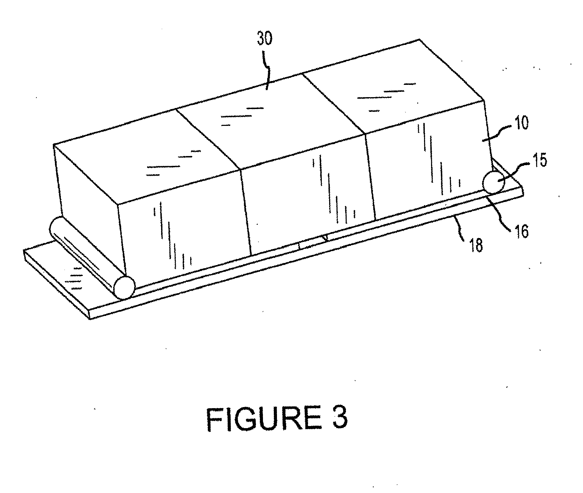

Figure 3 is a perspective view of the embodiment of figure 2 in a folded state. A lid 30 covers in this embodiment the batteries, processing device, motor and electronics, and is connected to the transverse plate 10. The legs 16, 17 are folded under the base plate 10 by means of the hinges 15, and the folded upper section rests on the back plate 18. This provides a very compact unit which easily may be stored and transported.

## Claims

1. Resuscitation system, comprising:
- chest compression devices to repeatedly compress the chest of a patient and thereafter causing or allowing the chest to expand,
- power supply devices, and
- a signal processor connected to the chest compression devices to control operation of the chest compression devices.

2. Resuscitation system according to claim 1,
**characterised in that** it comprises measuring devices for measuring characteristics of the resuscitation process, and that the signal processor is adapted to process input signals from the measuring devices.

3. Resuscitation system according to claim 2, where the measuring devices comprise a force sensor and/or a depth sensor.

4. Resuscitation system according to claim 2, where the measuring devices comprises a blood flow sensor.

5. Resuscitation system according to claim 2, where the measuring devices comprises a ventilation sensor.

6. Resuscitation system according to claim 1, comprising ventilation devices.

7. Resuscitation system according to claim 1, comprising defibrillator devices.

8. Resuscitation system according to claim 1,
**characterised in that** the power supply devices comprise power storage devices or devices for connection to power sources in an ambulance, in a hospital or in an external power storage device.

9. Resuscitation system according to claim 2, further comprising data storing devices connected to the measuring devices for storing values of the characteristics measured by the measuring device.

10. Resuscitation system according to claim 1, the system further comprising a user interface for providing information regarding the resuscitation to the operator.

11. Resuscitation system according to claim 7, the defibrillator device being an automatic external defibrillator (AED).

12. Resuscitation system according to claim 1, the system further comprising a support connected to the chest compression device.

13. Resuscitation system according to claim 1, the system comprising two separate parts.
